(19) Europäisches Patentamt / European Patent Office / Office européen des brevets

(11) **EP 4 079 368 B1**

(12) **EUROPEAN PATENT SPECIFICATION**

(45) Date of publication and mention of the grant of the patent:
**31.01.2024 Bulletin 2024/05**

(21) Application number: **22159571.3**

(22) Date of filing: **19.12.2017**

(51) International Patent Classification (IPC):
*A61N 1/05* (2006.01)    *A61N 1/36* (2006.01)

(52) Cooperative Patent Classification (CPC):
**A61N 1/36171; A61N 1/0551; A61N 1/36067; A61N 1/36157**

(54) **SENSORY INFORMATION COMPLIANT SPINAL CORD STIMULATION SYSTEM FOR THE REHABILITATION OF MOTOR FUNCTIONS**

MIT SENSORISCHEN INFORMATIONEN KONFORMES RÜCKENMARKSTIMULATIONSSYSTEM ZUR REHABILITATION DER MOTORISCHEN FUNKTIONEN

SYSTÈME DE STIMULATION DE LA MOELLE ÉPINIÈRE CONFORME AUX INFORMATIONS SENSORIELLES POUR LA RÉHABILITATION DES FONCTIONS MOTRICES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.12.2016 EP 16206606**

(43) Date of publication of application:
**26.10.2022 Bulletin 2022/43**

(62) Document number(s) of the earlier application(s) in accordance with Art. 76 EPC:
**17826212.7 / 3 558 448**

(73) Proprietor: **Ecole Polytechnique Fédérale de Lausanne**
**1015 Lausanne (CH)**

(72) Inventors:
• **FORMENTO, Emanuele**
**Berkley, 94709 (US)**
• **CAPOGROSSO, Marco**
**1003 Lausanne (CH)**
• **MICERA, Silvestro**
**1204 Genève (CH)**
• **COURTINE, Grégoire**
**1005 Lausanne (CH)**
• **MINASSIAN, Karen**
**1050 Vienna (AT)**

(74) Representative: **DTS Patent- und Rechtsanwälte Schnekenbühl und Partner mbB**
**Brienner Straße 1**
**80333 München (DE)**

(56) References cited:
**US-A1- 2012 083 709    US-A1- 2012 330 391**
**US-A1- 2013 289 664    US-A1- 2016 175 586**
**US-A1- 2016 271 413**

**Description**

**FIELD OF THE INVENTION**

The present invention refers to the field of spinal cord neuro-prostheses, in particular for motor disorders.

In particular, it refers to a system for stimulation of the spinal cord, more in particular for the rehabilitation of motor function in subjects with spinal cord injury or other motor disorders (e.g. consequent to stroke).

**BACKGROUND OF THE INVENTION**

Severe spinal cord injury (SCI) disrupts the communication between supraspinal centers and spinal circuits below the lesion, usually including those responsible for the generation of movement. The interruption of descending pathways abolishes the source of modulation and excitation that are essential to enable spinal circuits to be in a state in which they are able to produce movement, termed "functional state". For this reason, although largely anatomically intact, spinal cord circuits below the lesion remain in a state that is not permissive for standing and walking, termed "dormant state".

In humans SCI can be functionally complete (i.e. the subject cannot perform any functional movement below the level of the injury) or incomplete (i.e. the subject can control to some degree subsets of muscles below the injury). Even when a SCI is defined as complete from a functional point of view, some of the fibers connecting the brain to the spinal cord segments below the injury are spared. However, these are not enough to be able to recruit the surviving circuits below the lesion leading to paralysis.

Spinal cord stimulation (SCS) therapies are usually proposed to boost the excitability of spinal sensori-motor circuits and thus to facilitate the execution of motor commands.

In particular electrical neuromodulation of the spinal cord is used to provide excitation to the spinal circuits below the lesion, in order to replace the tonic descending drive from the missing brain inputs and set the spinal circuits to a "functional state" promoting restoration of movement. Indeed, with an increased excitation of the spinal circuits even the weak signals coming from the spared fibers, connecting the brain to the circuits below the injury, could be enough to generate a movement.

However, it has been shown in rats with complete spinal cord injury (Edgerton 2008, Courtine et al. 2009, Moraud et al 2016), that in the absence of brain inputs, surviving spinal circuits use sensory signals coming from the limbs as a source of motor control and modulation.

Therefore, sensory signals coming from the limbs, and in particular proprioceptive inputs which converge in muscle spindle feedback circuits, play a key role in the rehabilitation of movement after paralysis when using spinal cord stimulation (Takeoka et al. 2014, Moraud et al. 2016).

In this context, in the last decade it has been shown that electrical SCS of lumbar segments provides a strategy to reactivate spinal sensori-motor circuits, facilitating standing and walking in animals and to a limited degree in humans with SCI (Van den Brand et al., 2012; Angeli et al., 2014). After years of research, the first insights on the mechanisms underlying the recovery of locomotor functions with SCS are now emerging.

In particular, it is acknowledged that the main neural structures recruited by the stimulation are the large afferent fibers entering into the spinal cord from the dorsal roots (Rattay et al., 2000; Minassian et al., 2007; Capogrosso et al., 2013) and that the sensory information carried by these fibers, and especially the one coming from the muscle spindles proprioceptive fibers, plays an essential role in the recovery of locomotor movements after a spinal cord injury (Wernig and Müller, 1992; Takeoka et al., 2014).

In agreement with these data, it has been recently shown in rats, that the facilitation of locomotor movements mainly results from a synergistic interaction between the electrical stimulation protocols and the muscle spindles feedback circuits (Moraud et al., 2016). In particular, it has been shown that, in rats, standard stimulation protocols increase the overall firing rate of the afferent fibers without disrupting the naturally generated sensory feedback information carried and that this synergistic interaction is the mechanism underlying the known modulation of muscles activity with different stimulation frequencies (Moraud et al., 2014).

Animal experiments are essential in order to explore these mechanisms and consequently to develop innovative neuromodulation strategies that could be used in humans to provide patient specific therapies (Moraud et al., 2016; Wenger et al., 2016). In fact, the conservation of muscle spindle feedback circuits across mammals suggests that the same mechanisms may facilitate motor control in humans.

However, to date, results obtained in rats using continuous stimulation protocols did not fully translate to human patients.

Indeed, while SCS therapies can induce a full recovery of voluntary locomotion after severe SCI in rats (Van den Brand et al., 2012), in humans, only temporary facilitation of specific voluntary movements and static standing during continuous stimulation has been reported (Harkema et al., 2011, Angeli et al., 2014).

It has now been found that differences in fibers length and afferent firing properties between humans and rats dramatically affect the interaction of electrical stimulation with muscle spindle circuits neural activity, disrupting natural firing patterns and therefore limiting human translation of animal results.

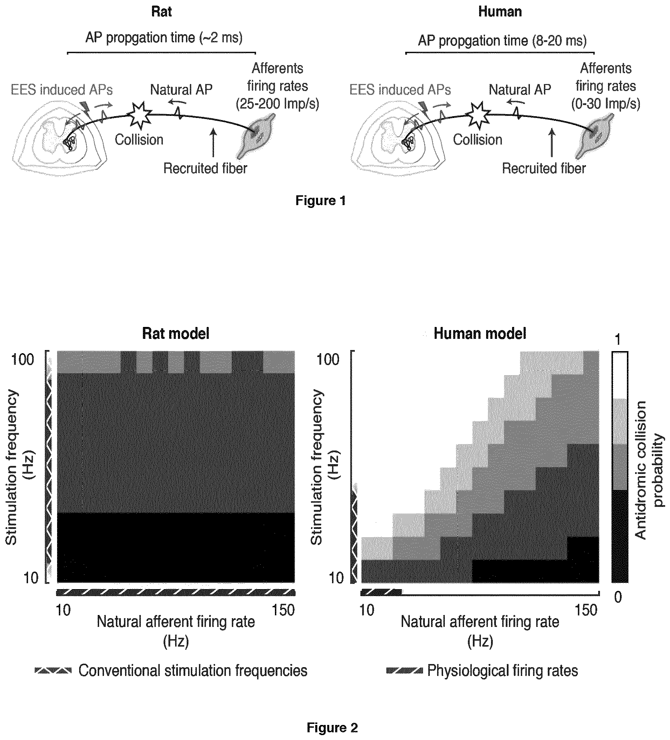

In particular, it has now been found that, in humans, even at relatively low SCS frequencies (e.g. 30 Hz), the stimulation completely erases the sensory information carried by the recruited fibers (see for example Figure 2) depriving the spinal circuits of meaningful signals that are needed to coordinate muscle activation in the absence of brain inputs.

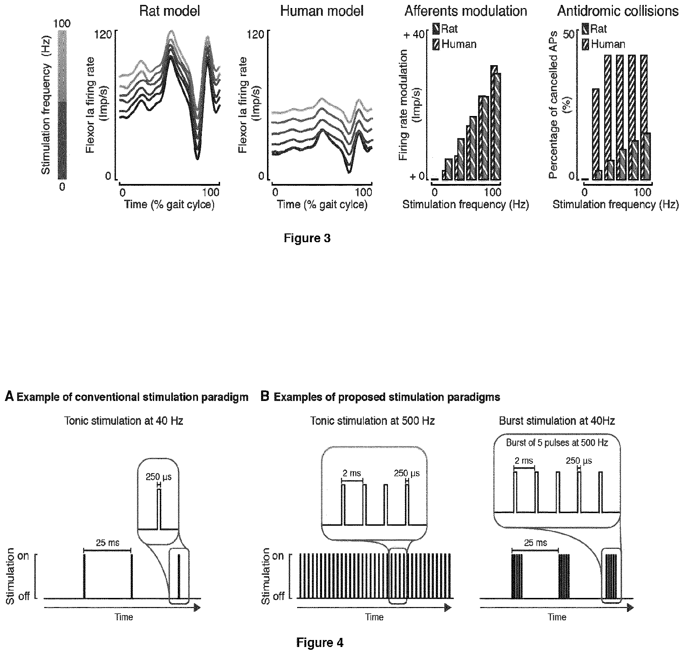

Indeed, it has been found that the increase in afferent modulation (i.e. the total number of action potentials per second in the afferent fibers that reaches the spinal cord) in humans, contrary to rats, is achieved at the expense of sensory information that gets completely erased in the recruited fibers (see for example Figure 3).

Therefore, while in rats SCS increases the firing rates of the afferent fibers by providing an additional tonic drive to the natural sensory information, in humans SCS increases the firing rates of the afferent fibers by *overriding* sensory information.

In view of the above results and the large amount of evidences showing the critical role of these sensory signals (Takeoka et al. 2014, Moraud et al 2016) it is evident that direct translation of SCS protocols developed in rodent models to the patients with motor disorders presents critical limitations.

It has now been found, and is an object of the present invention, a spinal cord stimulation system that overcomes these limitations by addressing the technological and neurophysiological requirements needed to achieve powerful modulation of the human spinal cord without disrupting sensory information.

Stimulation protocols currently used in the prior art are directly derived from results obtained in rodent experiments and use supra threshold amplitudes and frequencies ranging from 20 to 80 Hz.

For example, US2016121109 discloses a neuromodulation system wherein stimulation is delivered transcutaneously. Signals of 0.5-100 Hz are used with carrier frequencies of 5-10 kHz.

WO2016029159 discloses a method for improving bladder function delivering an epidural electrical stimulation with frequency of 1-100 Hz.

However, it has now been found that in humans sensory information in the recruited fibers is significantly disrupted by stimulation pulses interacting with sensory fibers at frequencies higher than 30 Hz, reducing the efficacy of such stimulation systems.

Therefore, there is still the need of a stimulation system able to overcome such deficiencies.

Some prior art documents discloses high-frequency stimulation of the spinal cord. For example, WO2012075195, WO2016064761, US2012016448. However, the systems disclosed in such documents are addressed to relieve pain in the patient and to this aim, frequencies in the order of kHz are used.

US2012/330391A1 discloses a SCS system including, inter alia, one or more (e.g. two) implantable stimulation leads, an implantable pulse source (IPG), and an external remote controller RC. The IPG is physically connected via one or more percutaneous lead extensions to the stimulation leads, which carry a plurality of electrodes arranged in an array. The IPG includes a pulse generation circuitry that delivers electrical stimulation energy in the form of a pulsed electrical waveform to the electrode array in accordance with a set of stimulation parameters. The stimulation leads are implanted within the spinal column of a patient. The system may be configured to deliver high frequency stimulation (e.g., above 100 Hz) having a narrow pulse width (e.g., below 20 μs). The stimulation amplitude may be adjusted up to motor threshold and then adjusted to a sub-motor level.

US2016/271413A1 discloses a system which is specifically designed for treatment of chronic pain in a patient by using multimodal stimulation of neural structures. The system comprises a signal generation module and one or more leads, configured for exposing glial cells and neurons simultaneously to a first electromagnetic stimulus and a second electromagnetic stimulus. In particular, the signal generation module can be configured for having an operating mode for providing a first electric signal with frequency between 200 Hz to 1,500 Hz to a first subgroup of electrodes, and a second electric signal with frequency lower than the first electric signal, such as between 20 Hz and 150 Hz, to a second subgroup of electrodes.

US2016/175586A1 discloses a system for delivery of sub-threshold spinal stimulation by using an implanted or epidural stimulator having one or more electrodes or electrode arrays, to produce one or more spontaneous voluntary movements in a patient. The document teaches, inter alia, an experimental design involving stimulation frequencies from 5 to 40 Hz using either a 210 or 450 μs pulse width, as well as a "locomotor pattern with epidural stimulation" involving delivery of epidural stimulation at 30-40 Hz along with task-specific sensory cues to generate locomotor-like patterns.

US2013/289664A1 discloses a system for delivery of electrical stimulation according to a spatial electrode movement pattern, in particular for treatment of chronic pain or other physiological dysfunctions of a patient. The system includes an implantable medical device (IMD) for delivering spinal cord stimulation (SCS) therapy and an external programmer. The IMD may be coupled to one or more leads. The IMD and/or the programmer may move electrical stimulation delivery between electrodes of the lead according to a spatial electrode movement pattern to achieve a desired therapeutic effect for the patient. A progression rate may indicate the frequency at which electrode combinations in the sequence are changed. The progression rate may generally be between 0.01 Hz and 10 Hz, such that a different electrode combination may be selected every 0.1 to 100 seconds.

US2012/083709A1 discloses a system and method for detecting intrathecal penetration, e.g. to implant leads proximate to the patient's spinal cord. In particular, the system may be used for implantation of a patient system for providing relief from chronic pain and/or other conditions, said patient system including a signal delivery system, which may be implanted within a patient typically at or near the patient's midline, and which is coupled to a pulse generator. The signal delivery device may include an elongated lead or lead body for providing therapy signals to the patient, e.g. provided with one or more electrodes or electrical contacts.

## SUMMARY OF THE INVENTION

It has now been found a system for spinal cord stimulation, in a subject with a spinal cord injury or other motor disorders, able to increase spinal excitability while minimizing the destructive interference with the sensory information, hence improving recovery of motor functions in said subject.

It is an object of the present invention a system for spinal cord stimulation of a subject according to claim 1. Further aspects and preferred embodiments are defined in the appended claims. The methods presented in the present description are provided for illustrative purposes only and do not form part of the present invention.

### Detailed description of the invention

### Definitions

Within the frame of the present invention, the following definitions are provided.

"Apparatus": means a component comprising one or more devices cooperating to provide a more complex function. Examples of apparatuses are a computer, a monitoring component. An apparatus can also be integrated in a system.

"System": means an ensemble of one or more apparatuses and/or devices cooperating to provide a more complex function.

"Operatively connected" means a connection capable of carrying data flow between two or more input and/or output data ports. The connection can be of any suitable type, a wired or a wireless connection.

"Processing device": means any device capable of elaborating data. Said device can be a processor, incorporated in a more complex apparatus or system, such as for example a computer.

"Subject" means an animal provided with spinal cord, in particular a mammal, more in particular a human.

A locomotion feature, or gait feature, is a kinematic parameter characterizing the gait cycle.

"Motor threshold amplitude" means the minimum amplitude necessary to induce a motor response with a single pulse of stimulation of the spinal cord.

"Subthreshold amplitude" means an amplitude not sufficient to induce a motor response with a single pulse of spinal cord stimulation.

"Destructive interference" defines the destructive interaction of the electrical stimulation with the sensory feedback information.

### Figures

Figure 1: Schematic representation of species specific factors influencing the occurrence probability of antidromic collisions in rats (left) and in humans (right). First, the propagation time required by an action potential (AP) to travel from the periphery to the spinal cord is around 2 ms in rats and between 8 to 20 ms in humans. Second, the natural firing rates of muscle spindle afferents during locomotion range between 25 Imp/s to 200 Imp/s in rats, while they rarely exceed 30 Imp/s in humans.

Figure 2: Occurrence probability of antidromic collisions between the spinal cord stimulation induced activity and the natural afferent firings, in rat and human afferent models. The rat (left) and human (right) models are characterized by the action potential propagation times, which are 2 and 16 ms respectively. For both models the antidromic collision probability is computed at different stimulation frequencies and at different firing rates of the afferent fibers.

Figure 3: Simulated effect of spinal cord stimulation on tibialis anterior afferents firing rates during locomotion, a comparison between rats and humans. The left panels report the rat and human afferents firing rates profiles during locomotion when different stimulation frequencies are used. The center-right panel reports the modulation induced in the afferent fibers for the different stimulation frequencies. The far right panel reports the percentage of sensory information cancelled by the stimulation at different frequencies.

Figure 4: (A) Example of stimulation signal used in conventional stimulation paradigms. A continuous train of pulses is delivered at 40 Hz. (B) Examples of proposed stimulation patterns. High frequency stimulation (500 Hz) is delivered either continuously (left) or as short bursts occurring at 40 Hz (right). Pulses width is 250 $\mu$s in both in conventional and in the proposed stimulation paradigms.

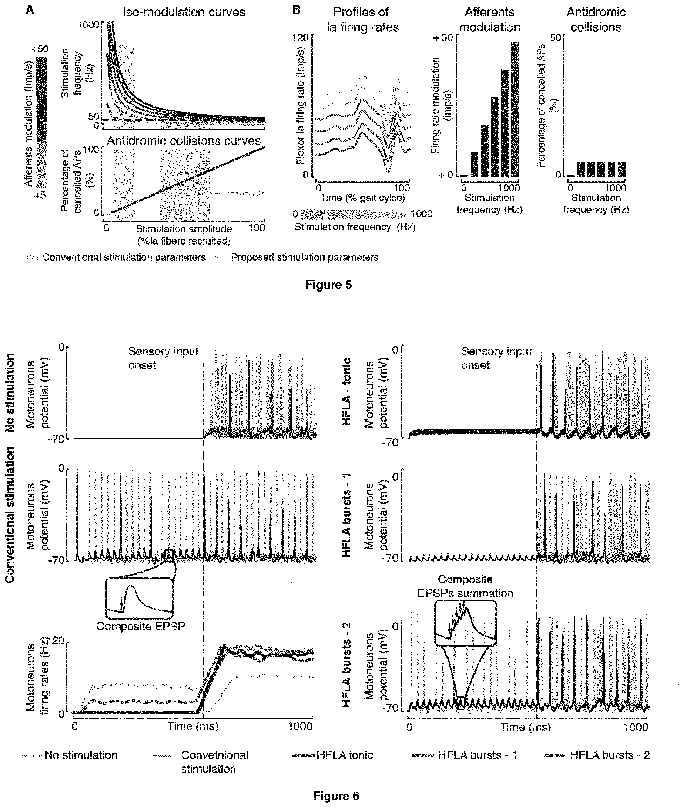

Figure 5: (A) Relationship between stimulation parameters and amount of antidromic collisions for different targeted modulations of the afferent fibers. The top panel report the iso-modulation curves, where each curve represents the stimulation frequency, as a function of the stimulation amplitude, necessary to induce a given modulation of the afferents firings. The shaded areas depict the set of parameters typical of conventional (grey area) and of the proposed stimulation protocols (grey checkered area). The bottom panel reports the antidromic collision curves showing the percentage of information cancelled by the stimulation, as a function of the stimulation amplitude, for the different modulations. The shaded areas depict the amount of antidromic collision induced by conventional (grey area) and by the proposed stimulation protocols (grey checkered area) (B) Simulated effect of the proposed stimulation protocol on the muscle spindle afferents firings during locomotion. The stimulation frequency is ranged from 0 Hz to 1000 Hz while the stimulation amplitude is kept constant at a value recruiting 5% of the Ia fibers. The left panel reports the estimated afferents firing rates profiles during gait. The center panel reports the overall modulation induced in the afferent fibers as a function of the stimulation frequency. The right panel reports the percentage of sensory information cancelled by the stimulation.

Figure 6: Simulated motoneurons modulations induced by different spinal cord stimulation protocols. The top-left panel represents the motoneuron activity induced only by afferent activity. The middle-left panel represents the motoneurons modulation induced by a standard spinal cord stimulation protocol: amplitude sufficient to recruit 40% of the Ia fibers and frequency of 40 Hz. The panels on the right represent motoneurons modulations induced by variations of the proposed stimulation paradigm: high-frequency low-amplitude stimulation (HFLA). The three protocols are all characterized by an amplitude sufficient to recruit 10% of the Ia fibers. Differences are on how the stimulation is delivered. The HFLA - tonic protocol is defined by a constant stimulation at 200 Hz, while HFLA burst 1 and 2 protocols are characterized by a stimulation delivered at bursts, of 3 and 5 pulses at 500 Hz respectively, occurring at 40 Hz. The zoom in boxes show a representative motoneuron response to a single pulse of conventional stimulation (left) and to a burst of high-frequency stimulation (right).

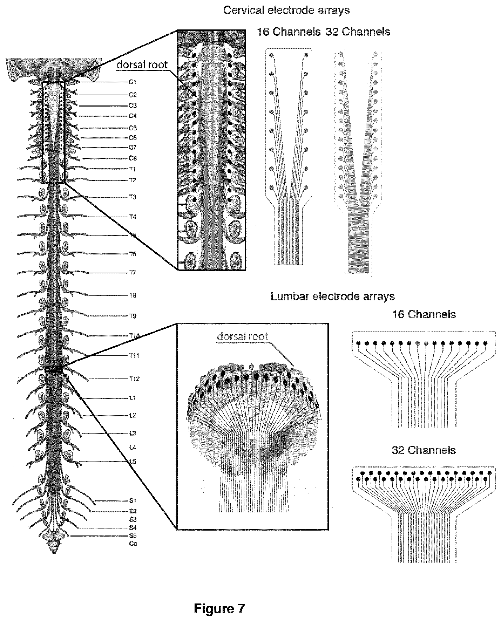

Figure 7: Proposed electrode designs. High density electrode array for cervical (top) and lumbar (bottom) spinal cord stimulation.

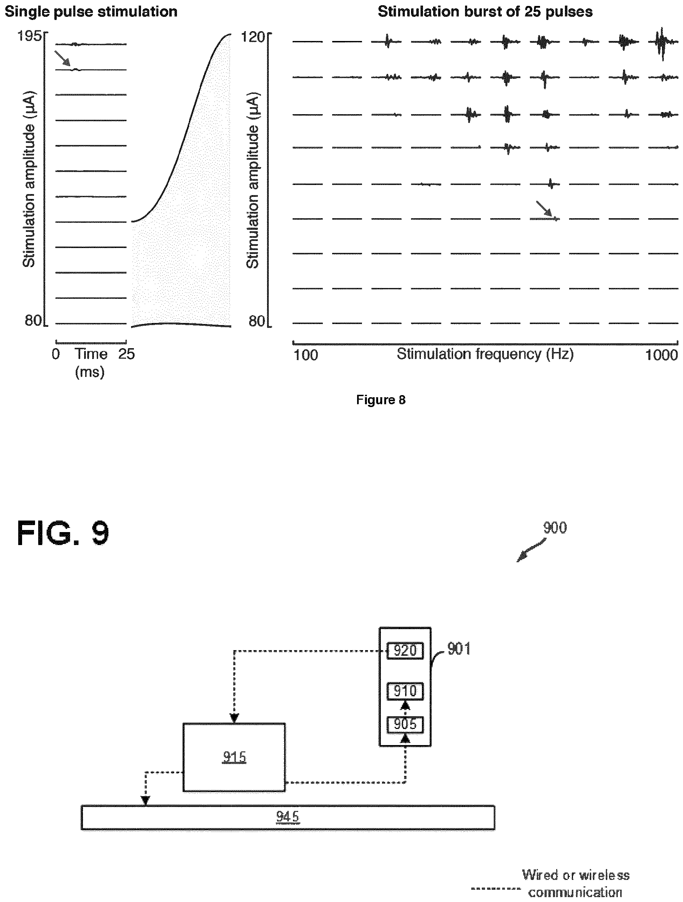

Figure 8: High frequency versus single pulse stimulation in rats. Representative responses of tibialis anterior induced by single pulses (left panel) or high-frequency bursts (right panel) of SCS for different stimulation parameters. Muscle responses to single pulses are induced only at amplitudes higher than 180 $\mu$A. Using high-frequency bursts, the lowest amplitude necessary to induce a response decreases to 95 $\mu$A when a stimulation frequency of 700 Hz is used. The responses recorded at these amplitudes are denoted by the arrows.

Figure 9: Depicts an example illustration of a system for spinal cord stimulation for a subject with a spinal cord injury or other motor disorder. Such a system may be able to increase spinal excitability while minimizing a destructive interference with sensory information, hence improving recovery of motor functions in said subject.

Turning now to Figure 9, example system 900 depicts the above-mentioned system for spinal cord stimulation. Specifically, system 900 may include a subject 901, to which the spinal cord stimulation may be applied. In one example of system 900, the system further comprises an implantable pulse generator (IPG) 905. IPG 905 may be operatively connected to deliver current pulses to one or more multi-electrode arrays 910. The one or more multi-electrode arrays 910 may be suitable to cover at least a portion of a spinal cord of subject 901, for applying a stimulation to dorsal roots of the spinal cord of subject 901. The one or more multi-electrode arrays 910 may be placed epidurally or subdurally, for example.

In one example of system 900, the system may further comprise a processing device 915, or controller 915. Processing device 915 may be operatively connected with IPG 905. In one example, processing device 915 may provide IPG 905 with stimulation parameters. As one specific example, such stimulation parameters provided to IPG 905 via processing device 915 may comprise particular frequency and amplitude simulation parameters elaborated according to algorithms that are a function of signals 920 acquired from subject 901. Such signals 920 may comprise kinematic, kinetic, and/or brain signals from subject 901, and signals 920 may further be utilized via processing device 915 to adjust said stimulation parameters in real-time. As one example, signals 920 may comprise electromyography (EMG) recordings from muscle, the muscle comprising muscle target by said stimulation. In some examples, signals 920 comprising kinematic signals may be obtained using a motion capture system (not shown). In some examples, such signals 920 may be obtained via one or more markers (not shown) positioned overlying anatomical landmarks of subject 901. In one example, processing device 915 may be included in IPG 905.

It may be understood that said stimulation may be applied via the one or more multi-electrode array(s) 910 on dorsal roots of the spinal cord of subject 901, in order to recruit afferent fibers of the spinal cord of subject 901. The one or more multi-electrode arrays 910 may comprise epidural, subdural, inraspinal and/or hook electrodes positioned on the dorsal roots of subject 901.

IPG 905 may be adapted to deliver multi-electrode array 910 stimulation characterized by a frequency comprise between 20 and 1200 Hz, and an amplitude comprised between 0.1 motor threshold amplitude and 1.5 motor threshold amplitude. For example, for motor threshold amplitude, it may be understood that it may comprise a minimum amplitude for inducing a motor response with a single pulse of spinal cord stimulation. In one example, the amplitude may comprise a subthreshold amplitude not sufficient to induce a motor response with a single pulse of spinal cord stimulation.

The stimulation provided to subject 901 may include stimulation characterized by a frequency between 20 and 1200 Hz. In one example, said stimulation is delivered in bursts of pulses, each burst occurring with a frequency preferably comprised between 20 and 50 Hz.

System 900 may allow for minimization of a destructive interference of the stimulation with sensory information, such that at least some of remaining descending commands to control locomotion may be preserved.

In one example, system 901 for applying electrical stimulation to the spinal cord of subject 901 may further comprise a training apparatus 945. The training apparatus 945 may include a treadmill in one example. In another example, training apparatus 945 may include a robot-assisted body-weight support. In still another example, training apparatus 945 may include a multidimensional trunk support system. Such a system 901 including training apparatus 945 may in some examples be used for facilitating upper limb movements, in particular reaching and grasping movements, in subject 901.

Accordingly, the system 900 of Figure 9 may comprise a system for spinal cord stimulation of a subject (e.g. 901) that achieves modulation of the spinal cord without disrupting sensory information. In other words, such a system may be able to increase spinal excitability in a subject (e.g. 901) while minimizing a destructive interference with sensory information, which may thus improve recovery of motor functions of said subject.

Such a system may enable a method for facilitating locomotor control in a subject (e.g. 901), comprising positioning on the spinal cord of said subject one or more multi-electrode arrays (e.g. 910) covering at least a portion of the spinal cord and able to apply a stimulation of dorsal roots of the spinal cord of said subject, and applying to said subject via an IPG (e.g. 905) stimulation characterized by a frequency comprised between 20 and 1200 Hz, more specifically between 200 and 1200 Hz, and an amplitude comprised of between 0.1 motor threshold amplitude and 1.5 motor threshold amplitude, through said multi-electrode array or arrays. In another example, said system may enable a method for facilitating upper limb movements in a subject in need thereof, using similar methodology to that described above for facilitating locomotor control in said subject.

In another example, the system 900 may comprise a system for stimulation of a spinal cord of a subject, the stimulation able to increase an excitability of spinal motor circuits of said subject, while minimizing an amount of a destructive interference of sensory feedback information. Such a system may include excitation of motoneurons of said subject, while minimizing stimulation of group 1a fibers. In one example of such a system, the stimulation may be provided in a continuous mode. In another example, the stimulation may be delivered as bursts of pulses, where each burst may comprise pulses having a frequency between 20 and 1200 Hz, and where each burst occurs at another frequency comprised between 5 and 80 Hz. In such an example, a number of pulses per burst may be variable, and may be selected in order to modulate an amount of delivered excitation. In such a system where the stimulation is provided as bursts of pulses, the stimulation may recruit afferent fibers less frequently, as compared to continuous stimulation. More specifically, in such a system an amount of homosynaptic depression of the 1a fibers may be reduced as compared to continuous stimulation.

In another example, the system 900 discussed above may enable a method for facilitating one or more of locomotion and/or upper limb motion control in a subject comprising providing to said subject electrical stimulation of a spinal cord of said subject via a programmable implantable pulse generator operatively connected to deliver current pulses to one or more multi-electrode arrays suitable to cover at least a portion of the spinal cord of said subject, where said electrical stimulation is applied to dorsal roots of the spinal cord, and where said IPG is adapted to deliver said multi-electrode array a stimulation characterized by a frequency comprised between 20 and 1200 Hz and an amplitude comprised between 0.1 motor threshold amplitude and 1.5 motor threshold amplitude, and where said electrical stimulation is provided either continuously or in short bursts. In such a method, recruitment of afferent fibers may be reduced when the electrical stimulation is provided in short bursts, as compared to when the electrical stimulation is provided continuously. In such a method, reduction of recruitment of afferent fibers when the electrical stimulation is provided in short bursts may further result in a lower amount of homosynaptic depression of afferent fibers (e.g. 1a fibers), as compared to when the electrical stimulation is provided continuously.

In one example, the system 900 may comprise a system for stimulating a spinal cord of a subject, while minimizing an amount of destructive interference resulting from said stimulation. In such an example, the stimulation may comprise high-frequency, low-amplitude (HFLA) electrical stimulation applied to the spinal cord of the subject. In one example of the system, the electrical stimulation excites motoneurons of said subject, while minimally recruiting group 1a afferent fibers. In one example, said electrical stimulation is provided in a continuous mode. In another example, said electrical stimulation is provided in a burst mode, the electrical stimulation delivered as bursts of pulses. In such an example where the electrical stimulation is delivered as bursts of pulses, each of said bursts may include a variable number of pulses. In the system, minimizing the amount of destructive interference resulting from said stimulation includes minimizing an interaction of the stimulation with sensory feedback information, the sensory feedback information comprising natural sensory information flowing into the spinal cord during execution of a movement, which may interact with the stimulation. Such examples will be further elaborated on in the continuing description provided below.

Conventional spinal cord stimulation protocols for promoting walking function, derived from animal models, use frequencies ranging from 20 to 50 Hz (Angeli et al., 2014; Danner et al., 2015) and amplitudes sufficient to induce leg muscle activation with each stimulation pulse.

Every pulse of electrical spinal cord stimulation elicits action potentials in the recruited fibers that propagate both orthodromically (i.e., running along the axon in its natural, normal direction) and antidromically (in the opposite direction). For the duration of the traveling time of an antidromic action potential toward the periphery, orthodromic activity in the same axon generated naturally in the periphery during the movements will be eliminated, a phenomenon known as antidromic collision. For this reason, part of the natural sensory information flowing into the spinal cord during movement execution could collide with antidromic action potentials elicited along the nerve by the stimulation, and thus being cancelled by the stimulation. This interaction of the electrical stimulation with the sensory feedback information is herein defined as destructive interference.

It has now been found that the differences in action potential propagation time and in the proprioceptive afferents firing rates among humans and rats drastically change the way SCS interacts with the sensory information. In particular, it was found that in rats the stimulation interacts in a synergistic manner with the natural sensory information for a broad range of stimulation frequencies, but in humans, even at relatively low SCS frequencies (e.g. 30 Hz), the stimulation completely erases any sensory information coming from the recruited fibers (Figure 1).

It has also been found that in humans, SCS at low frequencies (~20Hz) poorly modulates the natural afferent firing rates as the ratio between elicited and cancelled activity is close to one. When higher stimulation frequencies are used, the total number of action potentials per second in the afferent fibers that reaches the spinal cord can be increased. We hereafter termed this parameter "afferent modulation". However, the increase in afferent modulation in humans, contrary to rats, is achieved at the expense of sensory information that gets completely erased in the recruited fibers (Figure 3). Therefore, it has been found that while in rats SCS increases the firing rates of the afferent fibers by providing an additional tonic drive to the natural sensory information, in humans SCS increases the firing rates of the afferent fibers by overriding part of the sensory information.

Therefore, it has now been found that with the stimulation frequencies commonly used in SCS systems of the prior art, the sensory information in the electrically recruited fibers collides with the induced antidromic activity. Considering that to elicit a motor response through reflex circuits, approximately 40% of the la fibers need to be recruited, it is clear that these type of stimulation protocols drastically reduces the amount of sensory feedback information actually reaching the spinal cord. This sensory information is thought to be the source of motor control necessary to drive the recovery of locomotion.

It has now been found a system for stimulation of the spinal cord able to increase the excitability of spinal motor circuits while minimizing the amount of destructive interference.

The system of the invention is characterized by the application to the spinal cord of the subject of a stimulation characterized by high-frequency and low-amplitude (HFLA).

The proposed stimulation protocol is able to induce afferent modulation equal to that induced by conventional SCS protocols and at the same time to drastically reduce the amount of destructive interference.

The system of the invention provides strong motoneurons excitation while recruiting a minimal proportion of group la fibers.

In an embodiment, the stimulation is provided in a continuous mode.

In the system of the invention, high frequency stimulation is used to increase the excitation provided to the motoneurons by the recruitment of the afferent fibers. However, a continuous recruitment of the la fibers might elicit a strong post-activation depression at the motoneuron synapses that may reduce the excitation provided to the motor pools.

Therefore, in a preferred embodiment of the system of the invention, the stimulation is delivered as bursts of pulses, wherein each burst comprises pulses having a frequency according to the invention and each burst occurs at a frequency preferably comprised between 5 and 80 Hz, more preferably comprised between 20 and 50 Hz.

The number of pulses per burst is variable and selected in order to modulate the amount of delivered excitation. Compared to continuous stimulation, when the stimulation is delivered in bursts, the afferent fibers are recruited less frequently; therefore, the amount of homosynaptic depression of the Ia synapses is lower. Furthermore, the relatively long period of time between two consecutive bursts allows the Ia afferent motoneuron synapses to partially recover their efficacy.

For these reasons, a bursting stimulation results in a more effective neuromodulation.

As mentioned above, the main difference between conventional SCS and the stimulation system herein disclosed lays in how the excitation is provided to the motoneurons over time.

Conventional SCS protocols proposed to facilitate gait by recruiting the majority of Ia fibers, elicit strong synchronized excitations in the motor pools at every pulse. These excitations are normally supra threshold and for this reason they often result in a motor response (Figure 6, middle-left panel). When supplementary inhibitory or excitatory inputs are provided by other non recruited afferent fibers or by the remaining descending commands, the stimulation responses are either suppressed or amplified, thus allowing the generation of alternated movements such as locomotion. On the contrary, the system of the invention allows to recruit a reduced amount of fibers but at a higher frequency, thus inducing small depolarizations in the motoneuron membrane potentials that increase motor pools excitation by adding up in time. Depending on the stimulation parameters, stimulation can result in either motor responses or in a pure increase of spinal excitability (Figure 6, right panels). When stimulation is applied continuously, the result depends on stimulation frequency, amplitude and pulse width. When stimulation is applied in bursts of pulses, the result depends on stimulation frequency, burst frequency, number of pulses, amplitude and pulse width. The above mentioned parameters can therefore be modulated by the skilled person depending on the results one wants to obtain.

In particular, based on the stimulation parameters, in some embodiments a motor response can be obtained which is functionally very similar to that achieved with conventional SCS, but with the advantage that a significantly higher amount of sensory information is able to functionally modulate the sensorimotor circuits.

In other embodiments, based on the stimulation parameters, a pure increase of spinal excitability can be obtained, this meaning that no motor response occurs if no further input is provided to the spinal cord. This results in a more natural stimulation allowing the subject to have a more complete control of his/her muscular activity. This embodiment is particularly suitable for subjects with an incomplete lesion of the spinal cord or subjects at the end of a rehabilitation therapy.

Stimulation parameters

Stimulation parameters can be chosen among the followings: stimulation frequency, amplitude, pulse width, burst frequency and number of pulses. In particular, when stimulation is applied continuously, typical stimulation parameters are stimulation frequency, amplitude and pulse width. When stimulation is applied in bursts of pulses, typical stimulation parameters are stimulation frequency, burst frequency, number of pulses, amplitude and pulse width.

Algorithms can be used to elaborate the stimulation parameter settings.

In particular, stimulation amplitude can be elaborated on the basis of surface electromyography (EMG) recordings of the muscle targeted by the stimulation. EMG recordings can be performed according to the common general knowledge of the skilled person in the field.

For example, amplitude selection can be performed by recording surface EMG of the muscle targeted by the stimulation and searching the threshold amplitude on the basis of surface EMG responses.

Frequency is usually selected by visual inspection of the operator, according to the general knowledge in the field.

In conventional protocols, derived from animal models, wherein the frequency used is lower, the selection process of the stimulation amplitude is divided in two steps. First, the motor threshold is searched by sending single stimulation pulses at different amplitudes and monitoring the muscles EMG signals. Then, an amplitude approximately 1.2 times this threshold is used in order to ensure that every stimulation pulse elicits a muscle response.

In a preferred embodiment of the system of the invention a subthreshold amplitude is used. In this embodiment, no EMG responses are visible to estimate the amount of proprioceptive fibers recruited when a single pulse is sent. However, as shown in Figure 6, when high frequency stimulation is used, motor responses can be induced even when the amount of recruited fibers is low.

An exemplary algorithm used according to the present invention takes advantage of this property to automatically fine tune the stimulation amplitude and the frequency range required for the stimulation.

In particular, according to said algorithm high frequency stimulation bursts and surface EMG recordings are used to find the maximum stimulation frequency that can be followed by the afferent fibers and the minimum amplitude necessary to induce a motor response when this stimulation frequency is used. These stimulation parameters define both the maximum frequency that can be used to modulate spinal motor circuits excitability and the stimulation amplitude that leads to the recruitment of the smallest subset of proprioceptive fibers able to modulate the motoneuron membrane potential up to the excitation threshold. This amplitude, herein defined as high frequency motor threshold, can be selected for stimulation of the subject.

In an exemplary embodiment, an automatic search for these parameters is done in three steps. First, a predefined subthreshold amplitude is used to stimulate the spinal cord with a train of 10 pulses with inter-pulse period of 3 ms. This time period is chosen to be longer than the afferent fiber's absolute refractory period and for this reason every pulse is able to elicit an action potential in the recruited fibers. At the same time, surface EMG signals are recorded from the targeted muscles to asses whether the stimulation burst elicit a motor response. A standard optimization algorithm, as commonly known in the field, is then used to modify the stimulation amplitude until the lowest amplitude eliciting a motor response is found (motor threshold amplitude). In the second step of the algorithm, this amplitude is used to find the highest stimulation frequency that the afferent fibers are able to follow. In particular, a similar optimization approach is used to tune the inter-pulse period until the motor response reaches its maximum. Finally, the first step is repeated with the highest frequency possible in order to find the high-frequency motor threshold amplitude.

When stimulation is delivered at bursts, it is also necessary to define the number of pulses to be sent within each burst. For this purpose, a similar procedure as the one explained above can be performed. For example, the previously found amplitude and maximum frequency are used to stimulate the spinal cord with single bursts of different lengths, ranging between 2 and 10 pulses. At the same time, sEMG signals are recorded from the targeted muscles to asses the amplitude of the motor response. Finally, the burst that elicited the most powerful motor response is selected to define the highest number of pulses to use for each burst. Shorter bursts, together with the stimulation frequency, can be used to modulate the induced excitation.

According to the present invention the applicable stimulation frequency is comprised between 20 and 1200 Hz, preferably it is comprised between 200 and 1200 Hz, more preferably between 300 and 1000 Hz, even more preferably between 400 and 700 Hz.

Pulse-width is usually kept constant at a value comprised between 100 and 1000 $\mu$s, preferably at 300 $\mu$s.

Amplitude is comprised between 0.1 motor threshold amplitude and 1.5 motor threshold amplitude.

For motor threshold amplitude it is intended the minimum amplitude necessary to induce a motor response with a single pulse of stimulation of the spinal cord. The motor threshold amplitude can be easily identified case by case by the skilled person.

In a preferred embodiment, the amplitude is a subthreshold amplitude. For subthreshold amplitude it is intended an amplitude not sufficient to induce a motor response with a single pulse of spinal cord stimulation. Subthreshold amplitude can be easily identified case by case by the skilled person.

In a preferred embodiment, amplitude is comprised between 0.1 motor threshold amplitude and 0.99 motor threshold amplitude.

Exemplary methods to identify a suitable amplitude are disclosed above.

In an exemplary embodiment, amplitude can be comprised between about 0.6 and about 6 mA. Actual ranges and sub-ranges can vary from subject to subject.

When stimulation is delivered as bursts of pulses each burst comprises pulses having a frequency according to the invention and each burst occurs at a frequency preferably comprised between 5 and 100 Hz, more preferably comprised between 20 and 100 Hz, even more preferably between 20 and 80 Hz.

Electrode designs

Spinal cord stimulation facilitates the performance of locomotor functions by recruiting the large afferent fibers entering in the spinal cord from the dorsal roots. In particular, a selective stimulation of the relevant dorsal roots is required to develop personalized stimulation strategies and to maximize the therapeutic outcomes.

Therefore, multi-electrode arrays tailored to the anatomy of the dorsal roots are used in the present invention.

According to the interested spinal segment that one wants to stimulate multi-electrode arrays with different designs can be used.

In particular, when the lumbar spinal roots (L2, L3, L4, L5 and S1) are targeted a transversal positioning of the electrodes with respect to the spinal cord allows for a more selective stimulation of the dorsal roots.

Indeed, selective stimulation can be obtained by a transversal positioning of the electrodes that follows the topological organization of the rootlets around the lumbosacral levels.

Therefore, in a preferred embodiment, said multi-electrode array comprises electrodes arranged in a direction transverse relative to the spinal cord.

In particular, for selective stimulation of the lumbar dorsal roots the array is positioned at the sacral level in a direction transverse relative to the spinal cord so as to effectively stimulate a spinal dorsal root associated with a lumbar spinal segment.

Preferably, the electrodes of said array are arranged in one or more rows transversally surrounding at least a portion of the spinal cord.

The use of such array dramatically enhances the specificity of the stimulation, since single dorsal rootlets can be targeted reducing at the same time the recruitment of efferent fibers.

Such an array is preferably located at sacral level, more preferably under the T12 and L1 vertebrae.

Preferably, said array comprises a row comprising at least 5 electrodes. In a more preferred embodiment said multi-electrode array has between 3 and 40 electrodes, preferably between 5 and 32 electrodes.

In an even more preferred embodiment, said array comprises 16 transversal electrodes, preferably arranged in one row. In another preferred embodiment, it comprises 32 transversal electrodes, preferably arranged in two rows.

An exemplary embodiment of said array is depicted in Figure 7.

The use of high density multi-electrode arrays allows to provide a selective and homogeneous stimulation of the dorsal roots. This leads to an homogeneous recruitment of the desired spinal segments and thus to an enhanced selectivity of the stimulation.

This electrode arrangement is particularly preferred for selective stimulation of the L4, L5 and S1 roots. Since these roots are very close to the central portion of the cord at the sacral level, selective, limb specific stimulation is difficult to achieve with conventional means because of current spread in the cerebro-spinal fluid that might cause contralateral stimulation. With said electrode configuration selective stimulation of the L4 and L5 roots can be advantageously performed; consequently, selective stimulation of the Tibialis and Gastrocnemius muscles (innervated by the L4 and L5 roots) with no activation of the higher leg muscles is achieved.

In another preferred embodiment the multi-electrode array comprises multiple electrodes arranged in two or more columns disposed in a longitudinal direction relative to the spinal cord.

The use of such array in the system of the invention is particularly advantageous when target of sacral or cervical roots is desired. Indeed, in the sacral and cervical spinal portions the roots have a left/right symmetrical organization and they enter the cord at an angle comprised between 90 and 45 degrees for the cervical roots and between 45 and 60 degrees for the sacral roots.

The use of such longitudinal configuration of electrodes allows to selectively target only the desired dorsal roots thus providing high muscle specificity.

Preferably, in said array the electrodes are arranged in two columns, said columns being preferably placed on the opposite sides of the spinal cord.

In a preferred embodiment, said array comprises from 3 to 40 electrodes. In a preferred embodiment, it comprises 16 electrodes, preferably arranged in two parallel columns. In another preferred embodiment, it comprises 32 electrodes, preferably arranged in two parallel columns.

For the targeting of the cervical roots (C3, C4, C5, C6, C7, C8 and T1 roots) the array is preferably implanted under the C2-T1 vertebrae. An exemplary embodiment is depicted in Figure 7.

For the targeting of the sacral roots (L5 and S1 roots) the array is preferably implanted under the L5-S1 vertebrae.

According to the present invention, the electrodes of said multi-electrode array can be set as cathodes (-), anodes (+) or High impedance NULL.

Preferred stimulation sites are lumbar and sacral sites for lower limb stimulation and cervical sites for upper-limb stimulation. Lower limb stimulation is applied, for example, for facilitating standing and walking in a subject; upper-limb stimulation is applied, for example, for facilitating reaching and grasping.

More than one multi-electrode array can be used together in the system of the invention. Each array must be connected to the IPG.

In an embodiment, two multi-electrode arrays with two different electrode configurations according to the embodiments above described are used together so as to obtain an even more selective stimulation of the interested root and, subsequently, muscle.

For each electrode array, a combination of active sites can be used to stimulate the spinal cord. Such combination of active sites is herein defined as "electrode set". The suitable electrode set can be chosen by the skilled person according to specific conditions of the subject and according to the general knowledge in the field.

Location of electrodes can be determined by the skilled person based on the general knowledge of neuro-anatomical features of the subject to be treated.

Electrodes and arrays according to the invention can be manufactured with conventional materials and methods according to the general knowledge in the field.

Preferably, they can be manufactured using the Polydimethylsiloxane (PDMS)-based technology (Minev et al., 2015).

Surgical methods to insert and stabilize the spinal implant into the appropriate place, such as the epidural or subdural space, are known in the art.

Electrodes of the array can be epidural, subdural, intraspinal and/or hook electrodes. All such kinds of electrodes are already known in the art.

For example, laminectomies can be performed at vertebrae levels to create entry and exit points for the implant.

Electrophysiological testing can be performed intraoperatively to fine-tune positioning of electrodes.

Reference can be made, for example, to Courtine et al., 2009 and van den Brand, 2012.

Implantable pulse generator

In the system of the invention, one or more multi-electrode arrays are operatively connected to a IPG.

The IPG is a battery-powered micro-electronic device, implanted in the body, which delivers electrical stimulation.

Commercially available IPGs are suitable for the present invention.

An example of a commercially available IPG is the Eon Rechargeable IPG manufactured by Advanced Neuromodulation Systems, Inc..

The IPG used in the invention is able to deliver independent amounts of current to multiple electrodes simultaneously thus controlling independent current sources.

Each electrode may be set to function as cathode or anode or set to a high impedance state for a given pulse.

Each electrode of the multi-electrodes arrays of the invention targets single rootlets with monopolar stimulation.

In a preferred embodiment, said implantable pulse generator (IPG) is characterized by one or more of the following features:

- two or more independent stimulation channels, preferably between 8 and 32 stimulation channels;
- a stimulation frequency up to 1500 Hz;
- a high stimulation-amplitude resolution (preferably lower than 0.05 mA);
- real-time triggering capabilities (preferably lower than 30 ms) in order to change stimulation parameters according to external inputs such as, but not limited to, brain signals, kinematic signals and electromyography of muscles from the subject.

More in particular, a preferred IPG has 16 independent stimulation channels.

A preferred IPG has all the features listed above.

Preferably, the IPG has external wireless communication capabilities, more preferably with communication delays lower than 30 ms.

In a preferred embodiment, the IPG has an advanced customization of the stimulation protocols. This means that bursts and stimulation patterns can be modified according to the specific needs of the subject.

In an embodiment, the IPG has three operative modes: "tuning", "open-loop stimulation" and "phasic stimulation".

The "tuning" mode allows a processing device to control the stimulation by providing the stimulation parameters and settings.

The "open-loop stimulation" mode allows to provide a tonic stimulation of the spinal cord, delivered as bursts or continuously.

The "phasic stimulation" mode allows to time the stimulation according to signals from the patient, such as, but not limited to, brain signals, kinematic signals, electromyography of muscles.

Exemplary embodiments are disclosed below.

The "tuning" mode, needed for the automatic selection of the stimulation amplitude and frequency, allows an external computer to control the stimulation. Number of pulses to send, inter-pulse period, pulse-width and the stimulation amplitude are the variables that can be controlled from the external computer (or processing device). The "open-loop stimulation" mode can be used for a tonic stimulation of the spinal cord. In a preferred embodiment, up to 16 different active sites of the electrode array can be activated with different stimulation frequencies and amplitudes. The stimulation can be delivered either as bursts or continuously. Despite tonic stimulation has been the standard procedure for decades, recent results suggested that a phasic stimulation timed according to the different gait phases could be more effective (Wenger et al., 2016). For this reason, also a "phasic stimulation" mode is proposed. In this mode the IPG is controlled, for example wirelessly, from an external computer that times the stimulation on the different active sites according to kinematic events, such as foot-offs and foot-strikes, and/or according to signals coming from the subject, such as, but not limited to, brain signals, kinematic signals and electromyography of muscles.

In a preferred embodiment, stimulation settings can be modified within a maximum delay of, for example, 30 ms.

In both "open-loop stimulation" and "phasic stimulation" the IPG preferably allows for a high degree of customization of the stimulation patterns used to stimulate. A "tonic stimulation" mode can be selected in order to activate one or different active sites with a continuous train of pulses at a given frequency, pulse-width and amplitude. A further mode, named "burst stimulation", can be used to define more complex patterns of stimulation such as high-frequency stimulation delivered in bursts (see for example Figure 4 B - right panel). In this mode, 5 stimulation parameters need to be provided, namely: amplitude of stimulation, pulse-width, number of pulses within each burst, inter-bursts frequency and frequency of stimulation inside each burst.

Ranges of parameters can be obtained, for example using the algorithms above disclosed. Based on this information the skilled in the art, for example a physician, can select and fine tune the parameters according to the specific conditions of the subject and the effect of the stimulation on the subject. In all modalities the IPG preferably controls up to 16 different active sites with different stimulation parameters.

Finally, to allow for a smooth transition between different stimulation settings during the "phasic stimulation" operative mode, transitions between different stimulation amplitudes can be smoothed in time by setting an interval in which the amplitude linearly scale from the previous value to the new one.

The IPG provides the electrodes array with current pulses characterized by stimulation parameters which can be set, for example, according to the procedures and algorithms above disclosed.

In an embodiment, the system of the invention further comprises a processing device able to elaborate stimulation parameters and provide them to the IPG. Stimulation parameters can be elaborated from the processing device, for example, according to the procedures and algorithms above disclosed. A skilled in the art can make modifications to such algorithms as required by the specific needs and conditions of the patient according to the general knowledge in the field.

In an embodiment, the processing device receives information regarding the gait phase of the subject and elaborate suitable stimulation parameters which are provided to the IPG.

In another embodiment, the processing device receives information on the kinematic events of the subject, such us foot-offs and foot-strikes, and elaborate suitable stimulation parameters which are provided to the IPG.

In a further embodiment, the processing device receives information from the subject, such as brain signals, kinematic signals and electromyography of muscles, and elaborates suitable stimulation parameters which are provided to the IPG, preferably in real-time.

Medical uses

The system of the invention can be used for facilitating locomotor functions in a subject suffering from injured locomotor system, especially due to neuromotor impairment, in particular in a subject suffering from partial or total paralysis of limbs.

Therefore, it is an object of the invention the use of said system for facilitating locomotor functions in a subject suffering from a neuromotor impairment.

In particular, said neuromotor impairment can be partial or total paralysis of limbs.

Said neuromotor impairment may have been caused by a spinal cord injury, Parkinson's disease (PD), an ischemic injury resulting from a stroke, or a neuromotor disease as, for example, Amyotrophic Lateral Sclerosis (ALS) or Multiple Sclerosis (MS).

Preferably, the device is used for facilitating locomotor functions in a subject after spinal cord injury, Parkinson's disease (PD) or stroke.

The system of the invention can also be advantageously used for facilitating upper limb movements, in particular for facilitating reaching and grasping, in a subject with a neuromotor impairment.

Said neuromotor impairment may have been caused by a spinal cord injury, Parkinson's disease (PD), an ischemic injury resulting from a stroke, or a neuromotor disease as, for example, Amyotrophic Lateral Sclerosis (ALS) or Multiple Sclerosis (MS).

The following examples will further illustrate the invention.

**EXAMPLES**

*Methods*

**Computational model of the muscle spindle system and of its interactions with SCS**

To study the interaction between SCS and the natural flow of sensory information generated by peripheral sensory organs, we used an experimentally validated computational modeled of the muscle spindle system (Moraud et al, 2016). The model encompasses: (i) a model of muscles spindle afferent fibers that include realistic interactions between natural and SCS induced activity, (ii) a muscle spindle organ model coupled with a musculoskeletal model to estimate group Ia typical firing rates during locomotion and (iii) a model of alpha motoneurons and their excitatory input coming from Ia fibers. The model of the afferent fiber alone was used to study antidromic collisions and how their occurrence probability depends on the stimulation frequency, the length of the afferent fiber and the firing rate of the fiber. Estimates of the afferent firing rates during locomotion were used as input for groups of afferent fibers models to study the interaction between the natural flow of sensory information and SCS. Finally, the model of alpha motoneurons pools were used to estimate the effect of different SCS protocols on the motor output.

Muscle spindle afferents model

Group Ia muscle spindle afferents were modeled in NEURON (Hines and Carnevale, 1997) as custom artificial cells. These cells were modeled in order to account for realistic interactions between the natural flow of sensory information and the activity induced by electrical stimulation. Each fiber is characterized by a propagation time that defines the time required by every sensory action potential to travel from the muscle spindle to the synaptic terminals. In particular, propagation times of 2 and 16 ms were used respectively for rat and human afferent models.

We have updated the previous validated model by adding a component estimating the travelling times and collisions of action potentials along the sensory afferents. Action potential propagations were modeled for both natural action potentials carrying the sensory information and SCS induced activity. Whenever a natural action potential was simulated, an index representing the position of the action potential along the fiber was created and initialized at zero. Action potential propagation was modeled by increasing this index over time and by sending a synaptic input to all the neurons connected to the afferent fiber when the index reached the 'propagation time' value. To model SCS induced antidromic and orthodromic activity the same mechanism was used. In this case, two indexes were initialized at a value representing the position where the fiber is recruited, that is approximately at 1ms distance from the synaptic terminals. During time integration, one index, representing the action potential traveling orthodromically, was increased until a synaptic input was sent to the connected neurons when the 'propagation time' value was reached. At the same time, the second index, representing the action potential traveling antidromically, was decreased until it reached the opposite fiber ending at zero.

Coupling between natural and electrically induced neural activity

Coupling between the neural activity induced by the stimulation and the natural activity is highly non linear. Here, we considered two non linear properties of this interaction. First, neural activity induced by the stimulation was modeled only if the segment of fiber recruited by the stimulation was not already firing or under refractory period. A refractory period of 1 ms was used. Second, whenever antidromic activity traveling to the periphery encountered a natural action potential traveling centrally, antidromic collisions were modeled by simply canceling the two action potentials.

Estimate of group Ia muscle spindle afferents firings during locomotion

To estimate time profiles of firing rates of rat Ia afferent fibers during gait we used a muscle spindle model (Prochazka and Gorassini, 1998a, 1998b), described by equation (1), and a validated biomechanical model of the rat hindlimb (Johnson et al., 2011). We recorded joint trajectories during locomotion in healthy rats (n = 10 steps) and estimated muscle stretch profiles of flexor (tibialis anterior) and extensor (gastrocnemius medialis) muscles of the ankle through inverse kinematics. Fibers stretch and stretch velocity were linked to the envelope of EMG bursts to mimic the alpha-gamma linkage.

$$Rat\ Ia\ firing\ rate = 50 + 2 \bullet stretch + 4/3 \bullet sign(stretchVelocity) \bullet \left| stretchVelocity \right|^{0.6} + 50 \bullet EMGenv \quad (1)$$

Time profiles of human Ia afferent fibers during locomotion were predicted by multiplying the estimated rat firing rates by a factor of 0.4. This factor was estimated in order to achieve firings rates generally lower than 30 Hz, as human muscle spindles rarely exceed this firing rate (Prochazka, 1999). Despite this methodology does not provide an accurate estimate of human group Ia afferents firing rates dynamics in time during gait, for the purpose of this work we considered this approximation as satisfactory. Indeed, the estimated firing rates were only used to compute the amount of sensory information that is cancelled by the stimulation. This property is independent from the exact shape of the firing rates dynamics over time; instead it uniquely depends on the firing rate range (which is correctly predicted) and the conduction velocity of the fibers (which is correctly implemented in the model).

Motor pools of alpha motoneurons connected to group Ia afferents

To estimate the effect of different spinal cord stimulation protocols on motoneurons, we modeled a motor pool comprising 169 alpha motoneurons (Capogrosso et al., 2013; Jones and Bawa, 1997; McIntyre et al., 2002; Stienen et al., 2007) connected to the homologous pool of 60 Ia afferent fibers by excitatory synapses. The membrane potential of each motoneuron is described as a modified Hodgkin-Huxley model comprising sodium, potassium, calcium, and potassium-calcium gated ion channels (McIntyre et al., 2002). Motoneuron morphology consists of a $32 \pm 10$ mm diameter spherical soma connected to an electronic-equivalent dendritic tree of mammalian S type alpha motoneurons (Fleshman et al., 1988; Jones and Bawa, 1997), dendritic sizes adapted to match soma diameter, from cell S-type cell 35/4. The initial segment and efferent axon are implemented with dedicated membrane dynamics (Capogrosso et al., 2013; McIntyre et al., 2002). Excitatory synapses are modeled by an exponential function with reversal potential Esyn = 0 mV and decay time constant t = 0.5 ms. The conductance of these synapses was tuned to a mean EPSP amplitude of 212 mV (Harrison and Taylor, 1981), increased by 28% to mimic the heteronymous contribution of synergistic innervations (Scott and Mendell, 1976).

## Animals experiments

All procedures and surgeries were approved by the Veterinarian Office Vaud, Switzerland. The experiments were conducted on 5 adult female Lewis rats (200 g body weight, Centre d'Elevage R. Janvier).

## Surgical procedures

Procedures have been described in detail previously (Courtine et al., 2009; van den Brand et al., 2012). All interventions were performed under general anesthesia and aseptic conditions. Briefly, EMG electrodes were created by removing a small part ($1 mm notch) of insulation from a pair of Teflon-coated stainless steel wires inserted into the gastrocnemius medialis and tibialis anterior muscles of both hindlimbs. Stimulation electrodes (same type as EMG) were secured at the midline of the spinal cord at spinal levels L2 and S1 by suturing over the dura mater above and below the electrode. A common ground wire was inserted subcutaneously over the right shoulder. In the same surgery, the rats received a complete thoracic (T7) SCI necessary for further studies here not presented.

## Spinal cord stimulation and recordings

Spinal cord stimulation was delivered by an IZ2H stimulator and controlled by a custom RPvdsEx software implemented in a RZ2 BioAmp Processor unit (Tucker-Davis Technologies).
EMG signals were amplified and filtered online (10-5,000-Hz band-pass) by an AM-System, and recorded at 12.207 kHz by the RZ2 BioAmp Processor unit.

## Example 1

### Assessment of destructive interference in human spinal cord stimulation

Every pulse of electrical spinal cord stimulation elicits action potentials in the recruited fibers that propagate both orthodromically (i.e., running along the axon in its natural, normal direction) and antidromically (in the opposite direction). For the duration of the traveling time of an antidromic action potential toward the periphery, orthodromic activity in the same axon generated naturally in the periphery during the movements will be eliminated, a phenomenon known as antidromic collision. For this reason, part of the natural sensory information flowing into the spinal cord during movement execution could collide with antidromic action potentials elicited along the nerve by the stimulation, and thus being cancelled by the stimulation. We hereafter term this interaction of the electrical stimulation with the sensory feedback information as destructive interference.
The occurrence probability of these antidromic collisions is a function of three parameters: (i) the propagation time required by an action potential to travel from the sensory organ to the spinal cord, (ii) the fiber physiological firing rate and (iii) the SCS frequency. The first two parameters are species dependent and for this reason the amount of destructive interference differs between rats and humans (Figure 1). In particular, the propagation time is almost one order of magnitude higher in human than in rats because of the considerable difference in the fiber lengths. For instance, the time required for an action potential to travel from the gastrocnemius muscle spindles to the spinal cord in rat is around 2 ms, while in humans it amounts to approximately 16 ms. On the other hand, the physiological firing rates of rat muscle spindle afferents during locomotion range between 25 to 200 Hz, while the firing rates in humans rarely exceed 30 Hz (Prochazka, 1999).
To understand the effect of these parameters on the probability of the occurrence of antidromic collisions, we developed a model of muscles spindle afferent fibers that includes realistic interactions between natural and SCS induced activity. We used this model to compute the percentage of sensory information canceled by the stimulation, as a function of the action potential propagation time, the stimulation frequency and the natural firing rate. For this purpose, we integrated the dynamic of the fiber over 60 seconds and evaluated the amount of antidromic collisions for a broad range of parameters. We finally estimated the occurrence probability of antidromic collisions by averaging the result over 50 simulations initialized with different time delays between the first stimulation pulse and the first natural afferent activity.

The simulations revealed how the differences in action potential propagation time and in the proprioceptive afferents firing rates among humans and rats drastically change the way SCS interacts with the sensory information.

In particular, while we confirmed that in rats the stimulation interacts in a synergistic manner with the natural sensory information for a broad range of stimulation frequencies, we found that in humans, even at relatively low SCS frequencies (30 Hz), the stimulation completely erases any sensory information coming from the recruited fibers (Figure 2). We then evaluated the impact of this destructive interference during locomotion by modeling the effect of standard stimulation protocols on the afferent firing rates during gait (Figure 3). To this aim, we first estimated the firing rates profiles of tibialis anterior Ia afferents during locomotion, in rats and in humans. We then modeled a pool of 60 afferent fibers (Segev et al., 1990) and used the estimated firing rates to drive the natural activity of these cells over time. To simulate conventional protocols, we used a stimulation amplitude sufficient to recruit enough proprioceptive fibers (~40%) to trans-synaptically activate the motoneurons at every pulse of stimulation and frequencies ranging from 20 to 100 Hz. Different SCS frequencies were simulated in order to assess the effects of frequency modulation. The simulations showed that, in humans, SCS at low frequencies (~20Hz) poorly modulates the natural afferent firing rates as the ratio between elicited and cancelled activity is close to one. When higher stimulation frequencies are used, the total number of action potentials per second in the afferent fibers that reaches the spinal cord can be increased. We hereafter termed this parameter "afferent modulation". However, the increase in afferent modulation in humans, contrary to rats, is achieved at the expense of sensory information that gets completely erased in the recruited fibers (Figure 3). Therefore, while in rats SCS increases the firing rates of the afferent fibers by providing an additional tonic drive to the natural sensory information, in humans SCS increases the firing rates of the afferent fibers by overriding part of the sensory information.

Considering the importance of sensory information in guiding the recovery of motor functions after a spinal cord injury (Takeoka et al., 2014), these results point out an important limitation for the translation of SCS protocols developed in rodent models to the rehabilitation of patients with motor neuron disorders for both upper and lower limbs.

## Example 2

### Comparison between stimulation paradigm according to the invention and conventional stimulation protocols

Conventional spinal cord stimulation protocols for promoting walking function, derived from animal models, use frequencies ranging from 20 to 50 Hz (Angeli et al., 2014; Danner et al., 2015) and amplitudes sufficient to induce leg muscle activation with each stimulation pulse. As we showed before, with these stimulation frequencies the sensory information in the electrically recruited fibers collides with the induced antidromic activity. Considering that to elicit a motor response through reflex circuits, approximately 40% of the Ia fibers need to be recruited, it is clear that these type of stimulation protocols drastically reduces the amount of sensory feedback information actually reaching the spinal cord. This sensory information is thought to be the source of motor control necessary to drive the recovery of locomotion. For this reason, we hypothesize that a stimulation protocol that minimizes the amount of this destructive interference would improve the efficacy of SCS therapies for the rehabilitation of motor functions.

Muscle spindles primary afferent fibers make direct connections with all the motoneurons that innervate the muscle of origin (Mendell and Henneman, 1971; Segev et al., 1990). Therefore, even the stimulation of a single Ia fiber leads to an excitation of the whole homologous motor pool. Here, we take advantage of this property to design a new paradigm of SCS that increases the excitability of spinal motor circuits while minimizing the amount of destructive interference.

The stimulation protocols proposed here consist of using high-frequency low-amplitude (HFLA) stimulation, delivered either continuously or as short bursts, with each burst occurring at a frequency ranging between 20 and 50 Hz (Figure 4). Thanks to the broad connectivity of Ia fibers, decreasing the stimulation amplitude, hence lowering the percentage of recruited fibers, it is still possible to modulate the whole motor pool excitability. With respect to conventional protocols, the excitation provided to the motoneurons per pulse would be lower with decreasing SCS intensity, thus no motor responses would appear. However, exploiting the temporal summation of subsequent group Ia EPSPs at the motoneurons, an excitation equivalent to higher stimulation intensities can be induced over time by increasing the stimulation frequency. The recruitment of a smaller amount of fibers finally results in a lower amount of erased sensory information.

To show this concept, we estimated the relationship between stimulation frequency and amplitude when the induced afferent modulation is held constant, using the computational model we developed. We then computed the amount of sensory information erased by the stimulation according to the target modulation and the stimulation parameters (Figure 5 A).

The results illustrate how the proposed stimulation protocol is able to induce afferent modulation equal to that induced by conventional SCS protocols and at the same time to drastically reduce the amount of destructive interference.

To test the neuromodulation performance of this method, we then executed several simulations where we evaluated the effect of SCS on the afferent firings during locomotion when only 5% of the Ia fibers is stimulated at a frequency ranging from 0 to 1000 Hz (Figure 5 B).

The results confirmed how this stimulation paradigm is able to modulate the overall afferent firing rates with a minimal percentage of sensory information erased, in fact being approximately one order of magnitude lower than the one erased by conventional SCS protocols (Figure 3).

High frequency stimulation is used here to increase the excitation provided to the motoneurons by the recruitment of the afferent fibers. However, a continuous recruitment of the Ia fibers could elicit a strong post-activation depression at the motoneuron synapses that may significantly reduce the excitation provided to the motor pools. For this reason, we also propose a stimulation protocol consisting of high-frequency low-amplitude stimulation delivered as bursts, with each 'package of high-frequency stimulation' occurring at a lower frequency ranging between 20 and 50 Hz. The number of pulses per burst would be variable and selected, in order to modulate the amount of delivered excitation. Compared to continuous stimulation, with this protocol the afferent fibers are recruited less frequently; therefore, the amount of homosynaptic depression of the Ia synapses is lower. Furthermore, the relatively long period of time between two consecutive bursts allows the Ia afferent motoneuron synapses to partially recover their efficacy. For these reasons, a bursting stimulation results in a more effective neuromodulation.

As mentioned before, the main functional difference between conventional SCS paradigms and the ones proposed here lays in how the excitation is provided to the motoneurons over time. Conventional SCS protocols proposed to facilitate gait, by recruiting the majority of Ia fiber, elicit strong synchronized excitations in the motor pools at every pulse. These excitations are normally supra threshold and for this reason they often result in a motor response (Figure 6 middle-left panel). When supplementary inhibitory or excitatory inputs are provided by other non recruited afferent fibers or by the remaining descending commands, the stimulation responses are either suppressed or amplified, thus allowing the generation of alternated movements such as locomotion. On the other hand, by recruiting a reduced amount of fibers but at a higher frequency, the proposed stimulation protocols induce small depolarizations in the motoneuron membrane potentials that increase motor pools excitation by adding up in time.

Depending on the stimulation parameters, this type of stimulation results in either motor responses or in a pure increase of spinal excitability (Figure 6 right panels). The first case is functionally very similar to that achieved with conventional SCS, but with the protocol of the invention a significantly higher amount of sensory information is still able to functionally modulate the sensorimotor circuits. The latter case, instead, does not impose any muscle activity per se, but it facilitates the effects of sensory information and the remaining descending commands to control locomotion.

Therefore, it results in a more natural stimulation that allows the patients to have a more complete control of their muscular activity.

**Example 3**

**Effects of high-frequency SCS in rodents**

The disclosed stimulation paradigm relies on two independent mechanisms.

First, every Ia fiber has excitatory synaptic connections with all the motoneurons of the homonymous muscle. For this reason, even the recruitment of few fibers would convey excitation to the whole motor pool.

Second, excitatory post synaptic potentials (EPSPs) induced by the Ia fibers on the motoneurons are characterized by a duration at half amplitude of approximately 4 ms (Burke R. E, 1968). This suggests that the recruitment of few afferent fibers at high frequency would elicit strong depolarizations in the motoneurons because consecutive EPSPs will summate over time.

However, the maximum rate of EPSPs summation depends on the refractory period of the afferents that could be too long to allow for effective depolarization of the motoneurons. Moreover fast repetitive recruitment of Ia fibers would lead to homosynaptic depression of the synaptic terminals that will decrease the amplitude of the EPSPs over time. This may potentially limit the efficacy of our protocols. In order to test whether high frequency stimulation can be effectively used to excite the spinal motor pools at low amplitudes compared to conventional SCS we designed a dedicated animal experiment, which is described below.

Here we assessed whether with high frequency SCS is possible to evoke motor responses at a lower amplitude with respect to conventional protocols in SCI rats (n=5). We first searched for the lowest stimulation amplitude necessary to induce a motor response in the targeted muscles through a single pulse of stimulation over the L2 spinal level. For this purpose, we recorded muscle responses in both ankle flexor and extensor muscles while testing increasing stimulation amplitudes. The first amplitude evoking a response at least in one of the recorded muscles was considered as the threshold amplitude. Single pulses were used in order to mimic the conventional protocols where the summation of consecutive EPSPs on the motoneurons is negligible because of the relatively low stimulation frequency used.

We then searched the lowest amplitude necessary to induce a motor response when high frequency stimulation is used. We measured the muscles responses to bursts of stimulation for different stimulation amplitudes and frequency, with every burst consisting of a train of 25 pulses. We found that using high frequency stimulation the mean amplitude necessary to evoke a muscle response was lowered by 38% ± 10% with respect to the one required by single stimulation pulses. Furthermore, when bursts of stimulation were applied, the strongest responses were achieved with frequencies ranging between 600 and 700 Hz. In particular, the amplitudes of the motor responses raised with the increase of the stimulation frequency up to 700 Hz, while at 800 Hz they dropped drastically and then raised again at 900 Hz and 1000 Hz (Figure 8).

These results suggest that effective EPSPs summation can be induced in the motoneurons for a wide range of stimulation frequencies and that the maximum firing rate that the stimulated afferents fibers were able to follow was around 700 Hz. Indeed, the drop of the response at 800 Hz could be explained by the fact that these fibers were only recruited at 400 Hz as the refractory period was longer than the period between two consecutive pulses of stimulation.

This experiment proved that, in rats, high frequency stimulation can be effectively used in order to stimulate the spinal cord with a lower stimulation amplitude than those used in conventional stimulation protocols. Given the strong conservation of the studied sensorimotor circuits across mammals, it is reasonable to affirm that similar results are expected also in humans.

**References**

Angeli, C.A., Edgerton, V.R., Gerasimenko, Y.P., and Harkema, S.J. (2014). Altering spinal cord excitability enables voluntary movements after chronic complete paralysis in humans. Brain 137, 1394-1409.

Burke, R. E. Group Ia synaptic input to fast and slow twitch motor units of cat triceps surae. The Journal of physiology 196.3 (1968): 605.

Capogrosso, M. et al. A computational model for epidural electrical stimulation of spinal sensorimotor circuits. J. Neurosci. 33, 19326-19340 (2013).

Courtine, G. et al. Transformation of nonfunctional spinal circuits into functional states after the loss of brain input. Nat Neurosci 12, 1333-1342 (2009).

Danner, S.M., Hofstoetter, U.S., Freundl, B., Binder, H., Mayr, W., Rattay, F., and Minassian, K. (2015). Human spinal locomotor control is based on flexibly organized burst generators. Brain 138, 577-588.

Edgerton VR, Courtine G, Gerasimenko YP, Lavrov I, Ichiyama RM, Fong AJ, Cai LL, Otoshi CK, Tillakaratne NJ, Burdick JW, Roy RR. Training locomotor networks. Brain Res Rev. 2008 Jan;57(1):241-54. Epub 2007 Sep 16.

Fleshman, J. W. & Segev, I. Electrotonic architecture of type-identified alpha-motoneurons in the cat spinal cord. Journal of Neurophysiology (1988).

Harkema, S. J. et al. Effect of epidural stimulation of the lumbosacral spinal cord on voluntary movement, standing, and assisted stepping after motor complete paraplegia: a case study. The Lancet 377, 1938-1947 (2011).

Harrison, P. J. & Taylor, A. Individual excitatory post-synaptic potentials due to muscle spindle Ia afferents in cat triceps surae motoneurones. J. Physiol. (Lond.) 312, 455-470 (1981).

Hines, M. L. & Carnevale, N. T. The NEURON simulation environment. Neural Comput 9, 1179-1209 (1997).

Jones, K. E. & Bawa, P. Computer simulation of the responses of human motoneurons to composite 1A EPSPS: effects of background firing rate. Journal of Neurophysiology (1997).

Johnson, W. L., Jindrich, D. L., Zhong, H., Roy, R. R. & Edgerton, V. R. Application of a rat hindlimb model: a prediction of force spaces reachable through stimulation of nerve fascicles. IEEE Trans. Biomed. Eng. 58, 3328-3338 (2011).

McIntyre, C.C., Richardson, A.G., and Grill, W.M. (2002). Modeling the excitability of mammalian nerve fibers: influence of afterpotentials on the recovery cycle. J. Neurophysiol. 87, 995-1006.

Mendell, L.M., Henneman, E., 1971. Terminals of single Ia fibers: location, density, and distribution within a pool of 300 homonymous motoneurons. Journal of Neurophysiology 34, 171-187.

Minassian K, Persy I, Rattay F, Pinter MM, Kern H, Dimitrijevic MR. Human lumbar cord circuitries can be activated by extrinsic tonic input to generate locomotor-like activity. Hum Mov Sci 2007; 26: 275-95.

Minev, I.R., et al., Electronic dura mater for long-term multimodal neural interfaces. Science, 2015. 347(6218): p. 159-163.

Moraud, E. M. et al. Mechanisms Underlying the Neuromodulation of Spinal Circuits for Correcting Gait and Balance Deficits after Spinal Cord Injury. Neuron 89, 814-828 (2016).

Scott, J. G. & Mendell, L. M. Individual EPSPs produced by single triceps surae Ia afferent fibers in homonymous and heteronymous motoneurons. J. Neurophysiol. (1976).

Segev, I., Fleshman, J.W., Burke, R.E., 1990. Computer simulation of group Ia EPSPs using morphologically realistic models of cat alpha-motoneurons. Journal of Neurophysiology 64, 648-660.

Stienen, A. H. A., Schouten, A. C., Schuurmans, J. & van der Helm, F. C. T. Analysis of reflex modulation with a biologically realistic neural network. J Comput Neurosci 23, 333-348 (2007).

Prochazka, A., 1999. Quantifying proprioception. Prog. Brain Res. 123, 133-142.

Prochazka, A. & Gorassini, M. Ensemble firing of muscle afferents recorded during normal locomotion in cats. J. Physiol. (Lond.) 507 ( Pt 1), 293-304 (1998).

Prochazka, A. & Gorassini, M. Models of ensemble firing of muscle spindle afferents recorded during normal locomotion in cats. J. Physiol. (Lond.) 507 ( Pt 1), 277-291 (1998).

Rattay F, Minassian K, Dimitrijevic MR. Epidural electrical stimulation of posterior structures of the human lumbosacral cord: 2. quantitative analysis by computer modeling. Spinal Cord 2000; 38: 473-89.

Takeoka, A., Vollenweider, I., Courtine, G., Arber, S., 2014. Muscle Spindle Feedback Directs Locomotor Recovery and Circuit Reorganization after Spinal Cord Injury. Cell 159, 1626-1639. doi:10.1016/j.cell.2014.11.019

Van den Brand, R. et al. Restoring Voluntary Control of Locomotion after Paralyzing Spinal Cord Injury. Science 336, 1182-1185 (2012).

Wenger, N. et al. Spatiotemporal neuromodulation therapies engaging muscle synergies improve motor control after spinal cord injury. Nature Medicine 22, 138-145 (2016).

Wernig A, Müller S. Laufband locomotion with body weight support improved walking in persons with severe spinal cord injuries. Paraplegia 1992; 30: 229-38.

**Claims**

1. A system (900) for spinal cord stimulation of a subject (901) comprising:

   a) a programmable pulse generator (905),
   b) one or more multi-electrode arrays (910) suitable to cover at least a portion of the spinal cord of said subject (901) and configured to apply a selective stimulation to the dorsal roots of the spinal cord of said subject (901),

   wherein said pulse generator (905) is operatively connected to said multi-electrode array (910) and adapted to deliver to said multi-electrode array (910) a stimulation **characterized by** a frequency comprised between 20 Hz and 30 Hz, preferably of 30 Hz, and an amplitude comprised between 0.1 motor threshold amplitude and 1.5 motor threshold amplitude, and
   wherein the stimulation is delivered at bursts of pulses, each burst of pulses occurring with a frequency comprised between 5 Hz and 100 Hz, preferably between 5 Hz and 80 Hz, more preferably between 20 Hz and 80 Hz, even more preferably between 20 Hz and 50 Hz.

2. A system (900) according to claim 1, wherein said programmable pulse generator (905) is a programmable implantable pulse generator (IPG) (905).

3. The system (900) according to claim 1 or 2, wherein each burst of pulses includes a variable number of pulses, preferably 2 to 5 pulses.

4. The system (900) according to anyone of claims 1-3, wherein stimulation is **characterized by** a pulse width between 100 μs and 1000 μs, preferably of 250-300 μs.

5. The system (900) according to anyone of claims 1-4, wherein the amplitude is a subthreshold amplitude.

6. The system (900) according to anyone of claims 1-5 wherein the amplitude is comprised between 0.1 motor threshold amplitude and 0.99 motor threshold amplitude.

7. The system (900) according to anyone of claims 2-6 wherein said one or more multi-electrode arrays (910) comprises electrodes arranged in a direction transverse relative to the spinal cord, preferably wherein said electrodes are implanted at the sacral level of the spinal cord, preferably under the T12 and L1 vertebrae.

8. The system (900) according to claim 7 wherein said one or more multi-electrode arrays (910) comprise 16 electrodes, preferably arranged in one row transversal to the spinal cord, or 32 electrodes, preferably arranged in two rows transversal to the spinal cord.

9. The system (900) according to anyone of claims 2-6 wherein said one or more multi-electrode arrays (910) comprise electrodes arranged in two or more columns disposed in a longitudinal direction relative to the spinal cord.

10. The system (900) according to claim 9 wherein said one or more multi-electrode arrays (910) comprise 16 electrodes, preferably arranged in two parallel columns, or 32 electrodes, preferably arranged in two parallel columns, preferably wherein said two columns are disposed on the opposite sides of the spinal cord.

11. The system (900) according to anyone of claims 2-10 wherein said implantable pulse generator (IPG) (905) is **characterized by**:

    - two or more independent stimulation channels, preferably between 8 and 32 stimulation channels,
    - a stimulation frequency up to 1500 Hz;
    - a high stimulation-amplitude resolution, preferably less than 0.05 mA.

12. The system (900) according to anyone of the preceding claims further comprising a processing device (915) operatively connected with said pulse generator (905) to provide stimulation parameters to said pulse generator (905).

13. The system (900) according to claim 12, wherein said processing device (915) is configured and adapted to elaborate stimulation parameters according to algorithms that are functions of signals (920) acquired from the subject (901), preferably wherein said signals (920) include kinematic, kinetic and/or brain signals from the subject (901).

14. The system (900) according to claim 13, wherein said processing device (915) is further configured and adapted to adjust the stimulation parameters in real time based on detected signals (920) acquired from the subject (901).

15. The system (900) according to anyone of claims 2-14 wherein said one or more multi-electrode array (910) comprises epidural, subdural, intraspinal and/or hook electrodes positioned on the dorsal roots.

16. The system (900) of anyone of claims 1-15 for use for facilitating locomotor functions in a subject (901) suffering from a neuromotor impairment.

17. The system (900) of anyone of claims 1-15 for use for facilitating upper limb movements, in particular reaching and grasping movements, in a subject (901) with a neuromotor impairment.

**18.** A system (900) for use in restoring voluntary control of locomotion in a subject (901) suffering from a neuromotor impairment comprising

a) the system (900) of anyone of claims 1-15, and
b) an apparatus (945) selected from the group consisting of at least one of a treadmill or a robot-assisted body-weight support or a multidirectional trunk support system.

**Patentansprüche**

**1.** System (900) zur Rückenmarkstimulation eines Patienten (901), aufweisend:

a) einen programmierbaren Impulsgenerator (905),
b) eine oder mehrere Mehrfachelektrodenanordnungen (910), die dafür geeignet sind, mindestens einen Teil des Rückenmarks des Patienten (901) abzudecken, und die so konfiguriert sind, dass sie eine selektive Stimulation auf die Dorsalwurzeln des Rückenmarks des Patienten (901) ausüben,

wobei der Impulsgenerator (905) mit der Mehrfachelektrodenanordnung (910) funktionstechnisch verbunden und dafür geeignet ist, an die Mehrfachelektrodenanordnung (910) eine Stimulation abzugeben, die durch eine Frequenz zwischen 20 und 30 Hz, vorzugsweise 30 Hz, und eine Amplitude zwischen 0,1 Motorschwellenamplituden und 1,5 Motorschwellenamplituden gekennzeichnet ist, und
wobei die Stimulation in Impulsbündeln abgegeben wird, wobei jedes Impulsbündel mit einer Frequenz zwischen 5 Hz und 100 Hz erfolgt, vorzugsweise zwischen 5 Hz und 80 Hz, bevorzugter zwischen 20 Hz und 80 Hz und noch bevorzugter zwischen 20 Hz und 50 Hz.

**2.** System (900) nach Anspruch 1, wobei es sich bei dem programmierbaren Impulsgenerator (905) um einen programmierbaren implantierbaren Impulsgenerator (IPG) (905) handelt.

**3.** System (900) nach Anspruch 1 oder 2, wobei jedes Impulsbündel eine variable Anzahl von Impulsen enthält, vorzugsweise 2 bis 5 Impulse.

**4.** System (900) nach einem der Ansprüche 1-3, wobei die Stimulation durch eine Pulsbreite zwischen 100 μs und 1000 μs, vorzugsweise von 250-300 μs, gekennzeichnet ist.

**5.** System (900) nach einem der Ansprüche 1-4, wobei die Amplitude eine unterschwellige Amplitude ist.

**6.** System (900) nach einem der Ansprüche 1-5, wobei die Amplitude zwischen 0,1 Motorschwellenamplituden und 0,99 Motorschwellenamplituden liegt.

**7.** System (900) nach einem der Ansprüche 2-6, wobei die eine oder die mehreren Mehrfachelektrodenanordnungen (910) Elektroden aufweisen, die in einer Richtung quer zum Rückenmark angeordnet sind, vorzugsweise wobei die Elektroden auf der sakralen Ebene des Rückenmarks implantiert sind, vorzugsweise unter den Wirbeln T12 und L1.

**8.** System (900) nach Anspruch 7, wobei die eine oder die mehreren Mehrfachelektrodenanordnungen (910) 16 Elektroden aufweisen, vorzugsweise in einer Reihe quer zum Rückenmark angeordnet, oder 32 Elektroden aufweisen, vorzugsweise in zwei Reihen quer zum Rückenmark angeordnet.

**9.** System (900) nach einem der Ansprüche 2-6, wobei die eine oder die mehreren Mehrfachelektrodenanordnungen (910) Elektroden aufweisen, die in zwei oder mehr Spalten angeordnet sind, die in einer Längsrichtung relativ zum Rückenmark vorgesehen sind.

**10.** System (900) nach Anspruch 9, wobei die eine oder die mehreren Mehrfachelektrodenanordnungen (910) 16 Elektroden aufweisen, vorzugsweise in zwei parallelen Spalten angeordnet, oder 32 Elektroden aufweisen, vorzugsweise in zwei parallelen Spalten angeordnet, wobei die beiden Spalten vorzugsweise auf den entgegengesetzten Seiten des Rückenmarks angeordnet sind.

**11.** System (900) nach einem der Ansprüche 2-10, wobei der implantierbare Impulsgenerator (IPG) (905) **gekennzeichnet ist durch**:

- zwei oder mehr unabhängige Stimulationskanäle, vorzugsweise zwischen 8 und 32 Stimulationskanäle,
- eine Stimulationsfrequenz von bis zu 1500 Hz;
- eine hohe Stimulationsamplitudenauflösung, vorzugsweise weniger als 0,05 mA.

12. System (900) nach einem der vorhergehenden Ansprüche, darüber hinaus mit einer Verarbeitungsvorrichtung (915), die mit dem Impulsgenerator (905) funktionstechnisch verbunden ist, um dem Impulsgenerator (905) Stimulationsparameter bereitzustellen.

13. System (900) nach Anspruch 12, wobei die Verarbeitungsvorrichtung (915) so konfiguriert und angepasst ist, dass sie Stimulationsparameter gemäß Algorithmen ausarbeitet, die Funktionen von Signalen (920) sind, die von dem Patienten (901) kommend erfasst werden, wobei die Signale (920) vorzugsweise kinematische Signale, kinetische Signale und/oder Gehirnsignale von dem Patienten (901) umfassen.

14. System (900) nach Anspruch 13, wobei die Verarbeitungsvorrichtung (915) ferner so konfiguriert und angepasst ist, dass sie die Stimulationsparameter in Echtzeit auf der Grundlage von erfassten Signalen (920), die von dem Patienten (901) kommend erfasst wurden, einstellt.

15. System nach einem der Ansprüche 2-14, wobei die eine oder die mehreren Mehrfachelektrodenanordnungen (910) epidurale, subdurale, intraspinale und/oder Hakenelektroden umfassen, die an den Dorsalwurzeln positioniert sind.

16. System (900) nach einem der Ansprüche 1-15 zur Verwendung für die Erleichterung der Bewegungsfunktionen bei einem Patienten (901), der an einer neuromotorischen Störung leidet.

17. System (900) nach einem der Ansprüche 1-15 zur Verwendung für die Erleichterung von Bewegungen der oberen Gliedmaßen, insbesondere von Bewegungen zum Erreichen und Ergreifen, bei einem Patienten (901) mit einer neuromotorischen Störung.

18. System (900) zur Verwendung bei der Wiederherstellung der willentlichen Kontrolle der Fortbewegung bei einem Patienten (901), der an einer neuromotorischen Störung leidet, aufweisend

a) das System (900) nach einem der Ansprüche 1-15, und
b) eine Vorrichtung (945), die aus der Gruppe ausgewählt ist, die aus einem Laufband und/oder einer roboterunterstützten Körpergewichtsunterstützung und/oder einem multidirektionalen Rumpfunterstützungssystem besteht.


## Revendications

1. Système (900) pour la stimulation de colonne vertébrale d'un sujet (901) comprenant :

a) un générateur d'impulsions programmable (905) ;
b) une ou plusieurs batteries multi-électrodes (910) adaptées pour couvrir au moins une partie de la colonne vertébrale dudit sujet (901) et configurées pour appliquer une stimulation sélective aux racines dorsales de la colonne vertébrale dudit sujet (901) ;

dans lequel ledit générateur d'impulsions (905) est relié en fonctionnement à ladite batterie multi-électrodes (910) et conçu pour amener à ladite batterie multi-électrodes (910) une stimulation **caractérisée par** une fréquence comprise entre 20 Hz et 30 Hz, de préférence de 30 Hz, et une amplitude comprise entre une amplitude de seuil moteur de 0,1 et une amplitude de seuil moteur de 1,5 ; et
dans lequel la stimulation est délivrée selon des salves d'impulsions données, chaque salve d'impulsions se produisant à une fréquence comprise entre 5 Hz et 100 Hz, de préférence entre 5 Hz et 80 Hz, de façon davantage préférée entre 20 Hz et 80 Hz, de façon davantage préférée encore entre 20 Hz et 50 Hz.

2. Système (900) selon la revendication 1, dans lequel ledit générateur d'impulsions programmable (905) est un générateur d'impulsions implantable programmable (IPG) (905).

3. Système (900) selon la revendication 1 ou 2, dans lequel chaque salve d'impulsions comprend un nombre d'impulsions variable, de préférence de 2 à 5 impulsions.

**4.** Système (900) selon l'une quelconque des revendications 1 à 3, dans lequel la stimulation est **caractérisée par** une largeur d'impulsion comprise entre 100 µs et 1000 µs, de préférence de 250-300 µs.

**5.** Système (900) selon l'une quelconque des revendications 1 à 4, dans lequel l'amplitude est une amplitude infraliminaire.

**6.** Système (900) selon l'une quelconque des revendications 1 à 5 dans lequel l'amplitude est comprise entre une amplitude de seuil moteur de 0,1 et une amplitude de seuil moteur de 0,99.

**7.** Système (900) selon l'une quelconque des revendications 2 à 6, dans lequel lesdites une ou plusieurs batteries multi-électrodes (910) comprennent des électrodes agencées dans une direction transversale par rapport à la colonne vertébrale, de préférence dans lequel lesdites électrodes sont implantées au niveau sacré de la colonne vertébrale, de préférence sous les vertèbres T12 et L1.

**8.** Système (900) selon la revendication 7, dans lequel lesdites une ou plusieurs batteries multi-électrodes (910) comprennent 16 électrodes, de préférence agencées selon une ligne transversale par rapport à la colonne vertébrale, ou 32 électrodes, de préférence agencées suivant deux lignes transversales par rapport à la colonne vertébrale.

**9.** Système (900) selon l'une quelconque des revendications 2 à 6, dans lequel lesdites une ou plusieurs batteries multi-électrodes (910) comprennent des électrodes agencées suivant deux colonnes ou plus disposées dans une direction longitudinale par rapport à la colonne vertébrale.

**10.** Système (900) selon la revendication 9, dans lequel lesdites une ou plusieurs batteries multi-électrodes (910) comprennent 16 électrodes, de préférence agencées selon deux colonnes parallèles, ou 32 électrodes, de préférence agencées selon deux colonnes parallèles, de préférence dans lequel lesdites deux colonnes sont disposées sur les côtés opposés de la colonne vertébrale.

**11.** Système (900) selon l'une quelconque des revendications 2 à 10, dans lequel ledit générateur d'impulsions implantable (IPG) (905) est **caractérisé par** :

- deux canaux de stimulation indépendants ou plus, de préférence entre 8 et 32 canaux de stimulation ;
- une fréquence de stimulation pouvant atteindre 1500 Hz ;
- une résolution de stimulation-amplitude élevée, de préférence inférieure à 0,05 mA.

**12.** Système (900) selon l'une quelconque des revendications précédentes, comprenant en outre un dispositif de traitement (915) relié en fonctionnement audit générateur d'impulsions (905) de façon à fournir des paramètres de stimulation audit générateur d'impulsions (905).

**13.** Système (900) selon la revendication 12, dans lequel ledit dispositif de traitement (915) est configuré et conçu pour élaborer des paramètres de stimulation en fonction d'algorithmes dépendant de signaux (920) acquis à partir du sujet (901), de préférence dans lequel lesdits signaux (920) comprennent des signaux cinématiques, cinétiques et/ou cérébraux provenant du sujet (901).

**14.** Système (900) selon la revendication 13, dans lequel ledit dispositif de traitement (915) est en outre configuré et conçu pour ajuster les paramètres de stimulation en temps réel sur la base de signaux détectés (920) acquis à partir du sujet (901).

**15.** Système (900) selon l'une quelconque des revendications 2 à 14, dans lequel lesdites une ou plusieurs batteries multi-électrodes (910) comprennent des électrodes épidurales, subdurales, intraspinales, épidurales et/ou à crochet positionnées sur les racines dorsales.

**16.** Système (900) selon l'une quelconque des revendications 1 à 15, à utiliser pour faciliter les fonctions locomotrices chez un sujet (901) souffrant d'un trouble neuromoteur.

**17.** Système (900) selon l'une quelconque des revendications 1 à 15, à utiliser pour faciliter les mouvements d'un membre supérieur, notamment des mouvements visant à atteindre et attraper des objets, chez un sujet (901) souffrant d'un trouble neuromoteur.

18. Système (900) à utiliser pour restaurer un contrôle volontaire de mouvement locomoteur chez un sujet (901) souffrant d'un trouble neuromoteur, comprenant :

   a) le système (900) selon l'une quelconque des revendications 1 à 15 ; et
   b) un appareil (945) sélectionné dans le groupe constitué par moins un élément parmi un support de poids du corps assisté par tapis roulant ou robot ou un système de soutien du tronc pluridirectionnel.

**Figure 1**

**Figure 2**

Figure 3

Figure 4

EP 4 079 368 B1

Figure 5

Figure 6

Figure 7

**Single pulse stimulation**

**Stimulation burst of 25 pulses**

Figure 8

# FIG. 9

Wired or wireless
--------- communication

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2016121109 A
- WO 2016029159 A
- WO 2012075195 A
- WO 2016064761 A
- US 2012016448 A
- US 2012330391 A1
- US 2016271413 A1
- US 2016175586 A1
- US 2013289664 A1
- US 2012083709 A1

### Non-patent literature cited in the description

- **ANGELI, C.A. ; EDGERTON, V.R. ; GERASIMENKO, Y.P. ; HARKEMA, S.J.** Altering spinal cord excitability enables voluntary movements after chronic complete paralysis in humans. *Brain,* 2014, vol. 137, 1394-1409
- **BURKE, R. E.** Group Ia synaptic input to fast and slow twitch motor units of cat triceps surae. *The Journal of physiology,* 1968, vol. 196 (3), 605
- **CAPOGROSSO, M. et al.** A computational model for epidural electrical stimulation of spinal sensorimotor circuits. *J. Neurosci.,* 2013, vol. 33, 19326-19340
- **COURTINE, G. et al.** Transformation of nonfunctional spinal circuits into functional states after the loss of brain input. *Nat Neurosci,* 2009, vol. 12, 1333-1342
- **DANNER, S.M. ; HOFSTOETTER, U.S. ; FREUNDL, B. ; BINDER, H. ; MAYR, W. ; RATTAY, F. ; MINASSIAN, K.** Human spinal locomotor control is based on flexibly organized burst generators. *Brain,* 2015, vol. 138, 577-588
- **EDGERTON VR ; COURTINE G ; GERASIMENKO YP ; LAVROV I ; ICHIYAMA RM ; FONG AJ ; CAI LL ; OTOSHI CK ; TILLAKARATNE NJ ; BURDICK JW.** Training locomotor networks. *Brain Res Rev.,* 16 September 2007, vol. 57 (1), 241-54
- **FLESHMAN, J. W. ; SEGEV, I.** Electrotonic architecture of type-identified alpha-motoneurons in the cat spinal cord. *Journal of Neurophysiology,* 1988
- **HARKEMA, S. J. et al.** Effect of epidural stimulation of the lumbosacral spinal cord on voluntary movement, standing, and assisted stepping after motor complete paraplegia: a case study. *The Lancet,* 2011, vol. 377, 1938-1947
- **HARRISON, P. J. ; TAYLOR, A.** Individual excitatory post-synaptic potentials due to muscle spindle Ia afferents in cat triceps surae motoneurones. *J. Physiol. (Lond.),* 1981, vol. 312, 455-470
- **HINES, M. L. ; CARNEVALE, N. T.** The NEURON simulation environment. *Neural Comput,* 1997, vol. 9, 1179-1209
- **JONES, K. E. ; BAWA, P.** Computer simulation of the responses of human motoneurons to composite 1A EPSPS: effects of background firing rate. *Journal of Neurophysiology,* 1997
- **JOHNSON, W. L. ; JINDRICH, D. L. ; ZHONG, H. ; ROY, R. R. ; EDGERTON, V. R.** Application of a rat hindlimb model: a prediction of force spaces reachable through stimulation of nerve fascicles. *IEEE Trans. Biomed. Eng.,* 2011, vol. 58, 3328-3338
- **MCINTYRE, C.C. ; RICHARDSON, A.G. ; GRILL, W.M.** Modeling the excitability of mammalian nerve fibers: influence of afterpotentials on the recovery cycle. *J. Neurophysiol.,* 2002, vol. 87, 995-1006
- **MENDELL, L.M. ; HENNEMAN, E.** Terminals of single Ia fibers: location, density, and distribution within a pool of 300 homonymous motoneurons. *Journal of Neurophysiology,* 1971, vol. 34, 171-187
- **MINASSIAN K ; PERSY I ; RATTAY F ; PINTER MM ; KERN H ; DIMITRIJEVIC MR.** Human lumbar cord circuitries can be activated by extrinsic tonic input to generate locomotor-like activity. *Hum Mov Sci,* 2007, vol. 26, 275-95
- **MINEV, I.R. et al.** Electronic dura mater for long-term multimodal neural interfaces. *Science,* 2015, vol. 347 (6218), 159-163
- **MORAUD, E. M. et al.** Mechanisms Underlying the Neuromodulation of Spinal Circuits for Correcting Gait and Balance Deficits after Spinal Cord Injury. *Neuron,* 2016, vol. 89, 814-828
- **SCOTT, J. G. ; MENDELL, L. M.** Individual EPSPs produced by single triceps surae Ia afferent fibers in homonymous and heteronymous motoneurons. *J. Neurophysiol.,* 1976

- **SEGEV, I. ; FLESHMAN, J.W. ; BURKE, R.E.** Computer simulation of group la EPSPs using morphologically realistic models of cat alpha-motoneurons. *Journal of Neurophysiology,* 1990, vol. 64, 648-660
- **STIENEN, A. H. A. ; SCHOUTEN, A. C. ; SCHUURMANS, J. ; VAN DER HELM.** F. C. T. Analysis of reflex modulation with a biologically realistic neural network. *J Comput Neurosci,* 2007, vol. 23, 333-348
- **PROCHAZKA, A.** Quantifying proprioception. *Prog. Brain Res.,* 1999, vol. 123, 133-142
- **PROCHAZKA, A. ; GORASSINI, M.** Ensemble firing of muscle afferents recorded during normal locomotion in cats. *J. Physiol.,* 1998, vol. 507, 293-304
- **PROCHAZKA, A. ; GORASSINI, M.** Models of ensemble firing of muscle spindle afferents recorded during normal locomotion in cats. *J. Physiol.,* 1998, vol. 507, 277-291

- **RATTAY F ; MINASSIAN K ; DIMITRIJEVIC MR.** Epidural electrical stimulation of posterior structures of the human lumbosacral cord: 2. quantitative analysis by computer modeling. *Spinal Cord,* 2000, vol. 38, 473-89
- **TAKEOKA, A. ; VOLLENWEIDER, I ; COURTINE, G. ; ARBER, S.** Muscle Spindle Feedback Directs Locomotor Recovery and Circuit Reorganization after Spinal Cord Injury. *Cell,* 2014, vol. 159, 1626-1639
- **VAN DEN BRAND, R. et al.** Restoring Voluntary Control of Locomotion after Paralyzing Spinal Cord Injury. *Science,* 2012, vol. 336, 1182-1185
- **WENGER, N. et al.** Spatiotemporal neuromodulation therapies engaging muscle synergies improve motor control after spinal cord injury. *Nature Medicine,* 2016, vol. 22, 138-145
- **WERNIG A ; MÜLLER S.** Laufband locomotion with body weight support improved walking in persons with severe spinal cord injuries. *Paraplegia,* 1992, vol. 30, 229-38